# EUROPEAN PATENT SPECIFICATION

(11) **EP 1 961 420 B1**
(45) Date of publication and mention of the grant of the patent: **25.07.2012**
(21) Application number: 06834797.0
(22) Date of filing: 15.12.2006
(51) Int. Cl.: A61K 31/4188, A61P 13/12, C07D 491/107

(54) **AGENT FOR PREVENTION AND TREATMENT OF ACUTE RENAL FAILURE**
WIRKSTOFF ZUR PRÄVENTION ODER BEHANDLUNG VON AKUTEM NIERENVERSAGEN
AGENT DESTINE A LA PREVENTION ET AU TRAITEMENT DE L'INSUFFISANCE RENALE AIGUE

(30) Priority: 16.12.2005 JP 2005362579
(43) Date of publication of application: 27.08.2008
(73) Proprietor: Sanwa Kagaku Kenkyusho Co., Ltd, Nagoya-shi Aichi 461-8631 (JP); Hirosaki University, Hirosaki-shi, Aomori 0368560 (JP)
(72) Inventor: YAGIHASHI, Soroku, Hirosaki-shi, Aomori 0368560 (JP); OGASAWARA, Saori, Hirosaki-shi, Aomori 0368560 (JP); HIBI, Chihiro, Nagoya-shi, Aichi 4618631 (JP); KATO, Noriaki, Nagoya-shi, Aichi 4618631 (JP)
(74) Representative: Torggler, Paul Norbert
(86) International application number: PCT/JP2006/325054
(87) International publication number: WO 2007/069727

(56) References cited:
- EP-A2- 1 064 949
- WO-A1-2005/040182
- JP-A- 04 145 023
- JP-A- 2004 300 153
- SCHRIER ROBERT W ET AL: "Acute renal failure: definitions, diagnosis, pathogenesis, and therapy" JOURNAL OF CLINICAL INVESTIGATION, vol. 114, no. 1, July 2004 (2004-07), pages 5-14, XP002607745 ISSN: 0021-9738
- MCCORMACK A J ET AL: "The effects of aldose reductase inhibitors in diabetic nephropathy" JOURNAL OF DIABETIC COMPLICATIONS,, vol. 3, no. 1, 1 January 1989 (1989-01-01) , pages 18-26, XP023091878 ISSN: 0891-6632 [retrieved on 1989-01-01]
- DATABASE BIOSIS [Online] BIOSCIENCES INFORMATION SERVICE, PHILADELPHIA, PA, US; 1995, COWLEY BENJAMIN D JR ET AL: "Temporal alterations in regional gene expression after nephrotoxic renal injury" XP002607746 Database accession no. PREV199598179562 & COWLEY BENJAMIN D JR ET AL: "Temporal alterations in regional gene expression after nephrotoxic renal injury" JOURNAL OF LABORATORY AND CLINICAL MEDICINE, vol. 125, no. 2, 1995, pages 187-199, ISSN: 0022-2143
- YAGIHASHI SOROKU ET AL: "The role of the polyol pathway in acute kidney injury caused by hindlimb ischaemia in mice." THE JOURNAL OF PATHOLOGY APR 2010 LNKD- PUBMED:20112370, vol. 220, no. 5, April 2010 (2010-04), pages 530-541, XP002607747 ISSN: 1096-9896

## Description

### TECHNICAL FIELD

The present invention relates to novel medicinal use of hydantoin derivatives which are known as ARIs as defined in the claims.

### BACKGROUND ART

Systemic inflammatory response syndrome due to trauma, burns, pancreatitis, sepsis, or infection; disseminated intravascular coagulation syndrome; multiple organ failure; peripheral arterial occlusive disease; arteriosclerosis obliterans; and Crush syndrome are inflammatory diseases, and ischemic circulatory failure is initiated by the persistence or aggravation of inflammatory cytokinemia. As a result, vital organ damage and tissue injury occur, which evokes acute renal failure. That is, the above-mentioned disease is involved in vital prognosis, and currently, it significantly compromises patient's quality of life and is dreaded as a disease which shows a very high mortality rate. With reference to the treatment of the disease increased in severity, high dose steroid hormone therapy for the purpose of the inhibition of production of inflammatory cytokine and pharmacotherapy such as anticoagulant therapy for the purpose of the improvement of the systemic peripheral circulation have been performed. Alternatively, acute blood purification therapy, continuous renal support therapy, and plasma exchange for the purpose of the removal of causative substance have been performed. However, there is still no therapy to improve patient's prognosis and vital prognosis and the mortality rate is high.

Further, as a therapy for patients with end-terminal organ failure such as hepatic cirrhosis, organ transplantations such as liver transplantations have been performed around the world, but the postoperative management after the transplantation has been an important issue. In the circumstances, transplant results have been improved by various new immunosuppressive agents. However, for example, in liver transplantation, post-ischemia reperfusion after the transplantation may lead to acute renal failure. The frequency of hemodialysis is in the range of 2 to 21%, and it is said that the mortality rate in cases of dialysis is high.

From these reasons, there is an earnest desire for the advent of an effective and safe drug for the acute renal failure which is caused by the persistence or aggravation of systemic inflammatory response syndrome due to trauma, burns, pancreatitis, sepsis, or infection; disseminated intravascular coagulation syndrome; multiple organ failure; peripheral arterial occlusive disease; arteriosclerosis obliterans; and Crush syndrome. Further, in organ transplantation, there is an earnest desire for the advent of a drug to prevent and reduce the development of acute renal failure, which is posttransplantation complication.

Regarding (2S,4S)-6-fluoro-2',5'-dioxospiro[chroman-4,4'-imidazolidine]-2-carboxamide which was discovered in the present applicant, its development for diabetic neuropathy is being progressed as a compound which has potent aldose reductase inhibitory effects and having high safety even when taken over a long period.

Regarding the hydantoin derivative including (2S,4S)-6-fluoro-2',5'-dioxospiro[chroman-4,4'-imidazolidine]-2-carboxamide, use in diabetic neuropathy is described in Japanese Patent Application Laid-Open (JP-A) No. 61-200991, use in circulation disease is described in JP-A No. 4-173791, use in various diseases accompanied with aging is described in JP-A No. 6-135968, use in diabetic simple retinopathy is described in JP-A No. 7-242547, use in diabetic keratopathy is described in JP-A No. 8-231549, use in diabetic maculopathy is described in WO2005/072066, and use in severe diabetic retinopathy is described in WO2005/079792. However, the effectiveness of the hydantoin derivatives for use of an agent for prevention and treatment of acute renal failure has not been reported.
(Patent document 1) JP-A No. 61-200991
(Patent document 2) JP-A No. 4-173791
(Patent document 3) JP-A No. 6-135968
(Patent document 4) JP-A No. 7-242547
(Patent document 5) JP-A No. 8-231549
(Patent document 6) WO2005/072066
(Patent document 7) WO2005/079792

The article "Acute renal failure: definitions, diagnosis, pathogenesis, and therapie" by Robert W Schrier et al published in Journal of Clinical Investigation, Vol. 114, no. 1, July 2004 discloses that the treatment of acute renal failure involves renal replacement therapy and various combination therapies with vasodilator agents or anti-inflammatory agents. However, this article does not disclose a therapy to improve patient's prognosis and vital prognosis.

### DISCLOSURE OF THE INVENTION

### Problems to be solved by the Invention

The present invention was achieved in view of the above situations, and an objective of the present invention is to provide a preventive or therapeutic agent for acute renal failure. Particularly, the objective of the present invention is to provide an effective pharmaceutical agent for the acute renal failure which is caused by the persistence or aggravation of systemic inflammatory response syndrome due to particularly trauma, burns, pancreatitis, sepsis, or infection; disseminated intravascular coagulation syndrome; multiple organ failure; peripheral arterial occlusive disease; arteriosclerosis obliterans; and Crush syndrome; or posttransplantation complications.

### Means for Solving the Problems

First, the present inventors developed an experimental model of acute renal failure (hereinafter referred to as the present experimental model). In the present experimental model animal, creatine kinase (CK), urea nitrogen (BUN) and creatinine in the blood had increased significantly and the animal was presented with acute renal failure status which clinically requires renal dialysis. Subsequently, the present inventors evaluated (2S,4S)-6-fluoro-2',5'-dioxospiro[chroman-4,4'-imidazolidine]-2-carboxamide (generic name: Fidarestat) in order to show the efficiency of hydantoin derivatives using the present experimental models. As a result, it was found that an increase of CK, BUN and creatinine in blood was reduced and the onset of acute renal failure was completely suppressed in the Fidarestat administered group (the ischemia reperfusion and Fidarestat administered group).

That is, the present invention is an agent which comprises, as an active ingredient, a compound represented by the general formula: 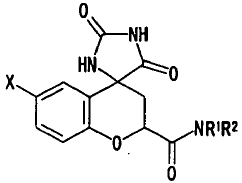 wherein X represents a halogen atom or a hydrogen atom, R1 and R2 independently represent a hydrogen atom or an optionally substituted C1 to C6 alkyl group, or R¹ and R², together with a nitrogen atom bound thereto, or optionally another nitrogen atom or an oxygen atom, are combined to form a 5- to 6-membered heterocycle, for use in the prevention or treatment of acute renal failure. Here, the halogen atom of X is preferably a fluorine atom. Further, as a C1-6 alkyl group, a C1-3 alkyl group is preferable and a methyl group is particularly preferable. A preferable example of the compound is (2S,4S)-6-fluoro-2',5'-dioxospiro[chroman-4,4'-imidazolidine]-2-carboxamide.

Examples of acute renal failure include acute renal failures resulting from ischemia or ischemia reperfusion.
That is, the acute renal failure which is caused by the persistence or aggravation of systemic inflammatory response syndrome due to particularly trauma, burns, pancreatitis, sepsis, or infection; disseminated intravascular coagulation syndrome; multiple organ failure; peripheral arterial occlusive disease; arteriosclerosis obliterans; and Crush syndrome; or posttransplantation complications can be exemplified.

### Effects of the invention

The present invention paves the way for drug therapy for the prevention or treatment of acute renal failure, particularly acute renal failure resulting from ischemia or ischemia reperfusion, namely, the acute renal failure which is caused by the persistence or aggravation of systemic inflammatory response syndrome due to particularly trauma, burns, pancreatitis, sepsis, or infection; disseminated intravascular coagulation syndrome; multiple organ failure; peripheral arterial occlusive disease; arteriosclerosis obliterans; and Crush syndrome; or posttransplantation complications. Further, the present invention causes no problems from a safety standpoint and provides a therapeutic agent which can be administered for a long-term period.

### BEST MODE FOR CARRYING OUT THE INVENTION

The present invention will be explained in more detail below.

The hydantoin derivative according to claim 1 can be orally administered for example, as tablets, capsules, powders, granules, liquids or syrups, or can be parenterally administered as injectables, infusions or suppositories, which were formed by conventional pharmaceutical manufacturing techniques. For pharmaceutical manufacturing, in the case of solid formulations, pharmaceutically acceptable excipients such as starch, lactose, purified white sugar, glucose, crystalline cellulose, carboxycellulose, carboxymethylcellulose, carboxyethylcellolose, calcium phosphate, magnesium stearate, gum arabic and the like can be used and, if necessary, lubricants, binders, disintegrating agents, coating agents, coloring agents and the like can be incorporated. In addition, in the case of liquid formulations, stabilizers, solubilizers, suspending agents, emulsifiers, buffers, preservatives and the like can be used. The dose is different depending on symptoms, age, administration methods, dosage forms and the like and, in the normal case, it is preferable that the compound described above is administered to an adult in a range of 0.5 to 300mg, preferably 1 to 150mg per day in terms of the present compound for consecutive days, once or a few times a day.

The above-described formulations of the present invention are for use in the prevention or treatment of acute renal failure. When pathological conditions responsible for acute renal failure are exemplified, examples thereof include systemic inflammatory response syndrome caused by trauma, systemic inflammatory response syndrome caused by burns, systemic inflammatory response syndrome caused by pancreatitis, systemic inflammatory response syndrome caused by sepsis, systemic inflammatory response syndrome caused by infection, disseminated intravascular coagulation syndrome, multiple organ failure, peripheral arterial occlusive disease, arteriosclerosis obliterans, Crush syndrome, or posttransplantation complications. The drug product of the present invention is especially effective for the acute renal failure which is caused by the persistence or aggravation of these conditions.

### Examples

### Test Example 1:

### Effect of Fidarestat on renal damage in ischemia reperfusion mice

### 1. Material and methods of pharmacological test

Sixteen- to twenty-week-old male C57BL/6 mice (Wild) were used in the experiment. The mice were divided into 4 groups, which were designated as sham-operation group (normal control group), ischemia operation group (ischemia reperfusion control group), ischemia operation + Fidarestat 40 mg/kg administration group (ischemia reperfusion/Fidarestat 40 mg/kg administration group), and ischemia operation + Fidarestat 150 mg/kg administration group (ischemia reperfusion/Fidarestat 150 mg/kg administration group), respectively. 40 or 150 mg/kg/day of Fidarestat was mixed in their diet and administered to the ischemia operation + Fidarestat administration groups 7 days before ischemia operation.

Plantar blood flow in these mice was measured with a laser blood flow meter under isoflurane anesthesia, and then the abdomen was incised. The abdominal aorta was exposed under a stereoscopic microscope and clipping was carried out at the portion distal to the renal artery bifurcation. Further, the right common iliac artery was also clipped and the right femoral artery was finally clipped. Thereafter, the abdomen was closed. The time of ischemia onset was set to the time when the femoral artery was clipped. From the time of ischemia onset, plantar blood flow was measured with a laser blood flow meter and the ischemia was confirmed. The clipping was released in order of the right femoral artery, right common iliac artery, and abdominal aorta in 3 hours from the ischemia onset and reperfusion was performed. At the time, the reperfusion status was confirmed with the laser blood flow meter. The reperfusion time was set to the time when the clipping of the aorta was released. The reperfusion was performed for 24 hours and the general condition was observed. Thereafter, heart blood was collected from the right atrium under isoflurane anesthesia. The collected heart blood was mixed with heparin and centrifuged at 3000g. Then, the supernatant was collected therefrom, which was frozen for preservation at -80 degrees C to use in a serologic test for CK, BUN, creatinine, and the like.

### 2. Results

Paralysis was observed in the ischemia reperfusion mice to which drug was not administered (ischemia reperfusion control group). The levels of creatine kinase (CK), urea nitrogen (BUN) and creatinine in blood were significantly increased compared with the normal control group and the mice presented with acute renal failure status which clinically requires renal dialysis. On the other hand, in the ischemia reperfusion/Fidarestat 40 mg/kg administration group and the ischemia reperfusion/Fidarestat 150 mg/kg administration group, the increase of CK in blood was reduced. Further, the increase of BUN and creatinine in blood was reduced to the level of the normal control group, and thus the onset of acute renal failure was completely suppressed.

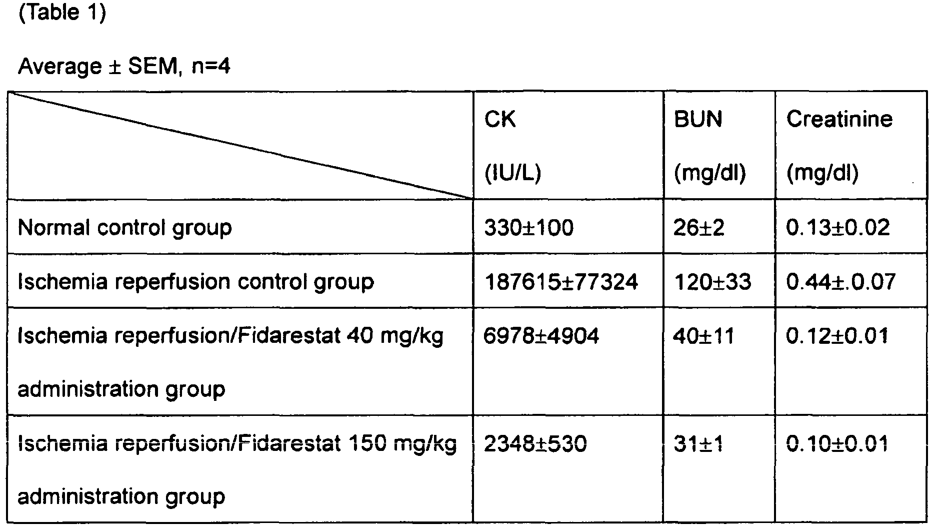

### Test Example 2:

### Effect of Fidarestat on renal damage in mice with LPS-induced inflammation

### 1. Material and methods of pharmacological test

Eight-week-old male CD-1 mice were used in the experiment. The mice were divided into 3 groups of normal control group (drug and LPS non-administration group), LPS control group (drug non-administration/LPS administration group), and LPS/Fidarestat administration group (Fidarestat and LPS administration group).

First, 10 mg/kg of Fidarestat (which was prepared with a solubilizing agent solution) was administered into the caudal vein of the Fidarestat administration group and the solubilizing agent solution was administered into the caudal vein of the drug non-administration group. After 5 minutes, Lipopolysaccharides (LPS: Escherichia coli; 0111: B4, Sigma) was prepared with a physiological salt solution, which was administered to the LPS administration group intraperitoneally at a concentration of 2 mg/kg and a physiological salt solution was administered to the LPS non-administration group intraperitoneally. After 24 hours, each of the groups of mice was anesthetized by ether inhalation and blood was collected from the abdominal vein using a heparinized syringe. Then, centrifugation was carried out under the following conditions: at 4 degree C, at 1,000 x g, for 10 min. Plasma was then obtained. Blood urea nitrogen (BUN) and creatinine were measured using an autoanalyzer (Hitachi). In this regard, as a solubilizing agent, NMDG (N-methyl-D-glucamine) was used.

### 2. Results

As shown in Table 2, the mice of the LPS control group had higher levels of BUN and creatinine compared with those of the normal control group. On the other hand, low levels of BUN and creatinine were observed in mice of the LPS/Fidarestat administration group. It is assumed that the mouse with LPS-induced inflammation exhibits pathological conditions clinically similar to renal damage which is caused by the aggravation of systemic inflammatory response due to sepsis, and it was shown that Fidarestat was effective for renal function impairment in the model with LPS-induced inflammation.

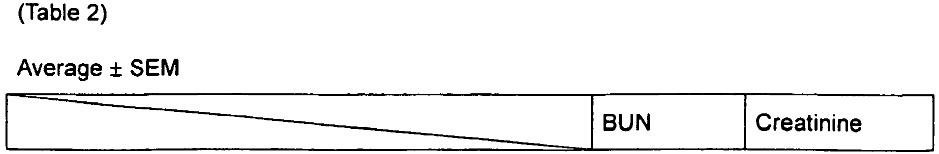

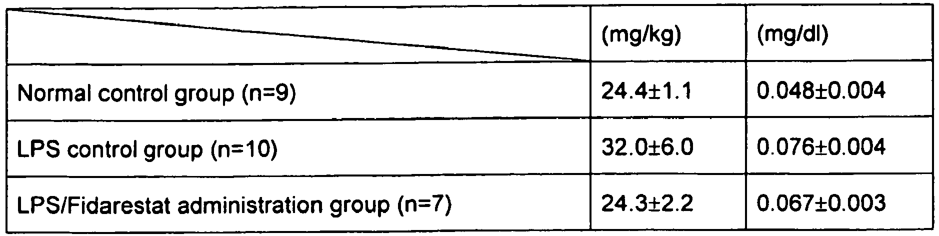

## Claims

1. An agent which comprises, as an active ingredient, a compound represented by the general formula: 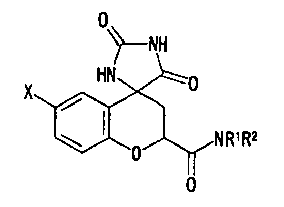 wherein X represents a halogen atom or a hydrogen atom, R1 and R2 independently represent a hydrogen atom or an optionally substituted C1 to C6 alkyl group, or R¹ and R², together with a nitrogen atom bound thereto, or optionally another nitrogen atom or an oxygen atom, are combined to form a 5- to 6-membered heterocycle, for use in the prevention or treatment of acute renal failure.

2. The agent for use in the prevention or treatment of acute renal failure according to claim 1, wherein the acute renal failure is the one resulting from ischemia or ischemia reperfusion.

3. The agent for use in the prevention or treatment of acute renal failure according to claim 1, wherein the acute renal failure is the one caused by the persistence or aggravation of systemic inflammatory response syndrome due to trauma, burns, pancreatitis, sepsis, or infection; disseminated intravascular coagulation syndrome; multiple organ failure; peripheral arterial occlusive disease; arteriosclerosis obliterans; and Crush syndrome; or posttransplantation complications.

4. The agent for use in the prevention or treatment of acute renal failure according to any one of claims 1 to 3, wherein the compound is (2S,4S)-6-fluoro-2',5'-dioxospiro[chroman-4,4'-imidazolidine]-2-carboxamide.

## Patentansprüche

1. Ein Mittel zur Anwendung für die Prävention oder Behandlung von akutem Nierenversagen, wobei das Mittel als aktiven Bestandteil eine Verbindung umfasst, die durch die allgemeine Formel 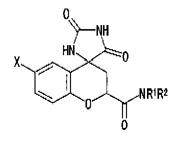 dargestellt ist, wobei X ein Halogenatom oder ein Wasserstoffatom darstellt, R1 und R2 unabhängig ein Wasserstoffatom oder eine wahlweise ersetzte C1 bis C6 Alkylgruppe darstellen, oder R¹ und R², gemeinsam mit einem daran gebundenen Stickstoffatom, oder wahlweise ein weiteres Stickstoffatom oder ein Sauerstoffatom, werden verbunden, um einen 5- bis 6-gliedrigen Heterozyklus zu bilden.

2. Das Mittel zur Anwendung für die Prävention oder Behandlung von akutem Nierenversagen gemäß Anspruch 1, wobei das akute Nierenversagen aus Ischemie oder ischemischer Reperfusion resultiert.

3. Das Mittel zur Anwendung für die Prävention oder Behandlung von akutem Nierenversagen gemäß Anspruch 1, wobei das akute Nierenversagen von Persistenz oder Verschlechterung von SIRS (Systemic Inflammatory Response Syndrome) aufgrund von Trauma, Verbrennungen, Pankreatitis, Sepsis oder Infektion; disseminiertem intravaskulärem Blutgerinnungssyndrom; multiplem Organversagen; peripherer arterieller Verschlusskrankheit; Arteriosklerosis obliterans; und Crush-Syndrom; oder postoperativen Komplikationen verursacht wird.

4. Das Mittel zur Anwendung für die Prävention oder Behandlung von akutem Nierenversagen gemäß einem der Ansprüche 1 bis 3, wobei die Verbindung (2S,4S)-6-Fluor-2',5'-Dioxospiro[Chroman-4,4'-Imidazolidin]-2-Carboxamid ist.

## Revendications

1. Agent qui comprend, en tant qu'ingrédient actif, un composé représenté par la formule générale : 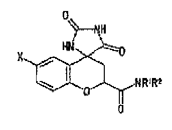 dans laquelle X représente un atome d'halogène ou un atome d'hydrogène, R¹ et R² représentent indépendamment un atome d'hydrogène ou un groupe alkyle en C₁ à C₆ facultativement substitué, ou R¹ et R², conjointement avec un atome d'azote auquel ils sont liés, ou facultativement un autre atome d'azote ou un atome d'oxygène, sont combinés pour former un hétérocycle à 5 à 6 chaînons, pour une utilisation dans la prévention ou le traitement d'une insuffisance rénale aiguë.

2. Agent pour une utilisation dans la prévention ou le traitement d'une insuffisance rénale aiguë selon la revendication 1, dans lequel l'insuffisance rénale aiguë est celle qui résulte d'une ischémie ou d'une ischémie-reperfusion.

3. Agent pour une utilisation dans la prévention ou le traitement d'une insuffisance rénale aiguë selon la revendication 1, dans lequel l'insuffisance rénale aiguë est celle qui est provoquée par la persistance ou l'aggravation d'un syndrome de réponse inflammatoire systémique dû à un traumatisme, des brûlures, une pancréatite, une sepsie ou une infection ; d'un syndrome de coagulation intravasculaire disséminée : d'une défaillance d'organes multiples ; d'une maladie occlusive artérielle périphérique ; d'une artériosclérose oblitérante ; et d'un syndrome d'écrasement ; ou de complications post-transplantation.

4. Agent pour une utilisation dans la prévention ou le traitement d'une insuffisance rénale aiguë selon l'une quelconque des revendications 1 à 3, dans lequel le composé est le (2S,4S)-6-fluoro-2',5'-dioxospiro[chroman-4,4'-imidazolidine]-2-carboxamide.
